# EUROPEAN PATENT SPECIFICATION

(11) **EP 3 442 549 B1**
(45) Date of publication and mention of the grant of the patent: **20.03.2024**
(21) Application number: 17715744.3
(22) Date of filing: 06.04.2017
(51) Int. Cl.: A61K 35/745, A23L 33/135, A61P 3/00, A61P 21/00

(54) **BIFIDOBACTERIA FOR INCREASING LEAN BODY MASS**
BIFIDOBAKTERIEN ZUR ERHÖHUNG VON MAGERER KÖRPERMASSE
BIFIDOBACTÉRIES POUR AUGMENTER LA MASSE MAIGRE D'UN CORPS

(30) Priority: 14.04.2016 EP 16165376
(43) Date of publication of application: 20.02.2019
(73) Proprietor: International N&H Denmark ApS, 2800 Kongens Lyngby (DK)
(72) Inventor: STENMAN, Lotta, 48210 Kotka (FI); LAHTINEN, Sampo, 08350 Lohja (FI)
(74) Representative: International N&H EMEA
(86) International application number: PCT/EP2017/058203
(87) International publication number: WO 2017/178315

(56) References cited:
- WO-A1-2016/020488
- WO-A1-2016/020495
- WO-A2-2010/146568

## Description

### FIELD OF THE INVENTION

This invention relates to the use of a composition comprising a bacterium of the genus *Bifidobacterium,* particularly, but not exclusively, a bacterium of *the Bifidobacterium animalis ssp. lactis* (strain) 420 (B420), and one or more prebiotics and/or fibres. This invention also relates to food products, dietary supplements and pharmaceutically acceptable formulations containing said bacterium.

### BACKGROUND OF THE INVENTION

Lean Body Mass is a component of body composition, calculated by subtracting body fat weight from total body weight. The percentage of total body mass that is lean is typically between 60-90%. Lean Body Mass is mostly muscle.

Muscle plays a central role in whole-body protein metabolism by serving as the principal reservoir for amino acids to maintain protein synthesis in vital tissues and organs in the absence of amino acid absorption from the gut and by providing hepatic gluconeogenic precursors.

Furthermore, altered muscle metabolism plays a key role in the genesis, and therefore the prevention, of many common pathologic conditions and chronic diseases. Nonetheless, the maintenance of adequate muscle mass, strength, and metabolic function has rarely, if ever, been targeted as a relevant endpoint of recommendations for dietary intake (Am. J. Clin. Nutr., September 2006, vol., 84, n.3, 475-482).

A grave change associated with human ageing is progressive decline in muscle mass, a downward spiral that may lead to decreased strength and functionality.

In 1989, it was proposed the term "sarcopenia" to describe this age-related decrease of muscle mass. Sarcopenia is a condition characterized by loss of skeletal muscle mass and function. Although it is primarily a disease of the elderly, its development may be associated with conditions that are not exclusively seen in older persons. Sarcopenia is a syndrome characterized by progressive and generalized loss of skeletal muscle mass and strength and it is strictly correlated with physical disability, poor quality of life and death (Santilli et al., Clinical Cases in Mineral and Bone Metabolism 2014; 11(3): 177-180).

Geriatric syndromes are common, complex and costly impair health in older individuals. Geriatric syndromes result from incompletely understood interactions of disease and age on multiple systems, producing a constellation of signs and symptoms. Delirium, falls and incontinence are examples of geriatric syndromes.

Sarcopenia is prevalent in older populations. Sarcopenia has multiple contributing factors: the ageing process over the life course, early life developmental influences, less-than-optimal diet, bed rest or sedentary lifestyle, chronic diseases and certain drug treatments . Sarcopenia represents an impaired state of health with a high personal toll-mobility disorders, increased risk of falls and fractures, impaired ability to perform activities of daily living, disabilities, loss of independence and increased risk of death (Age Ageing, 2010 Jul; 39(4); 412-423).

While sarcopenia is mainly observed in older people, it can also develop in younger adults, as is likewise the case for dementia and osteoporosis. In some individuals, a clear and single cause of sarcopenia can be identified. In other cases, no evident cause can be isolated. Thus, the categories of primary sarcopenia and secondary sarcopenia may be useful in clinical practice. Sarcopenia can be considered "primary" (or age-related) when no other cause is evident but ageing itself, while sarcopenia can be considered "secondary" when one or more other causes are evident. In many older people, the aetiology of sarcopenia is multi-factorial so that it may not be possible to characterise each individual as having a primary or secondary condition. This situation is consistent with recognising sarcopenia as a multifaceted geriatric syndrome.

Prado et al., J. Parental and Enteral Nutr., 2014*,* argues the importance of lean body mass and its relation to mortality and morbidity.

Ruiz et al., BMJ, 2008*,* describes the link between muscular strength and mortality.

The primary treatment for sarcopenia is exercise. Specifically, resistance training or strength training - exercise that increases muscle strength and endurance with weights or resistance bands - has been shown to be useful for both the prevention and treatment of sarcopenia. However, for elderly people, or people physically impaired, it may be difficult, or even impossible to perform the exercises necessary to increase the lean body mass or to minimize the consequences of the loss of the lean body mass.

The present invention seeks to provide a solution to the problems of the prior art.

WO2010/146568 discloses new uses of Bifidobacteria and food products, feed products, dietary supplements and pharmaceutical formulations containing them. The bacteria are suitable for the treatment of diabetes, obesity and related conditions, metabolic syndrome, insulin resistance, and impaired glucose metabolism and consequences thereof, lowering tissue inflammation, treating hepatitis, myositis and cardiovascular conditions.

WO2016/020495 discloses a maternal nutrition composition comprising myo-inositol and probiotics to address specific nutritional needs of women desiring to get pregnant, of pregnant and lactating women and of their offspring. The combination of myo-inositol and one or more probiotic to a subject may minimize excessive fat accretion (fat storage) and increase lean muscle mass in said subject. The probiotics comprise a combination of Lactobacillus and Bifidobacterium, such as strains Lactobacillus rhamnosus GG and BB12.

WO2016/020488 discloses myo-inositol and one or more probiotic for use to improve insulin sensitivity and/or treat or prevent type II diabetes and/or to prevent CVD and/or a condition associated with a low insulin sensitivity in a subject. Probiotic strains Lactobacillus rhamnosus GG and Bifidobacterium lactis BB-12 are disclosed.

### SUMMARY OF THE INVENTION

The invention concerns a composition comprising a bacterium of the genus *Bifidobacterium* of the *Bifidobacterium animalis ssp. lactis* (strain) 420 (B420), and one or more prebiotics and/or fibres, for therapeutic use in treating and preventing sarcopenia by increasing lean body mass in a mammal.

In another aspect, the invention concerns the non-therapeutic use of a composition comprising a bacterium of the genus *Bifidobacterium* or a mixture thereof, and one or more prebiotics and/or fibres, for increasing lean body mass in a mammal, wherein the bacterium of the genus *Bifidobacterium* is the *Bifidobacterium* of the *Bifidobacterium animalis ssp. lactis* (strain) 420 (B420).

### Advantages

It has surprisingly been found by the present inventors that treatment with a composition comprising a bacterium of *Bifidobacterium animalis ssp. lactis* (strain) 420 (B420), and one or more prebiotics and/or fibres, shows an increase in lean body mass in mammals. This confers the potential for bacterium of the genus *Bifidobacterium* or a mixture thereof, especially the *Bifidobacterium* of the *Bifidobacterium animalis ssp. lactis* (strain) 420 (B420) combined with one or more prebiotics and/or fibres to be useful in treating and/or preventing a number of diseases related to the loss of lean body mass.

### DETAILED DISCLOSURE OF THE INVENTION

### Bacteria

The bacterium used in the present invention is selected from a bacterium of the genus *Bifidobacterium* or a mixture thereof. Preferably the *Bifidobacterium* to be used in the present invention is a *Bifidobacterium* which is generally recognised as safe and, which is preferably GRAS approved. Generally recognized as safe (GRAS) is an American Food and Drug Administration (FDA) designation that a chemical or substance added to food is considered safe by experts, and so is exempted from the usual Federal Food, Drug, and Cosmetic Act (FFDCA) food additive tolerance requirements.

In one embodiment, the present invention relates a composition comprising a bacterium of the genus *Bifidobacterium* or a mixture thereof, and one or more prebiotics and/or fibres.

In another embodiment, the present invention relates a composition comprising a bacterium of the genus *Bifidobacterium* or a mixture thereof, and one or more prebiotics and/or fibres, for use in increasing lean body mass in a mammal.

In another embodiment, the present invention related to the use of a composition comprising a bacterium of the genus *Bifidobacterium* or a mixture thereof, and one or more prebiotics and/or fibres, for use in increasing lean body mass in a mammal.

In a further embodiment, the present invention relates to a method for increasing the lean body mass comprising administering to a mammal a composition comprising a bacterium of the genus *Bifidobacterium* or a mixture thereof, and one or more prebiotics and/or fibres, wherein the administration of the composition increases the lean body mass in the mammal.

In yet a further embodiment, the present invention related to the use of a composition comprising a bacterium of the genus *Bifidobacterium* or a mixture thereof, and one or more prebiotics and/or fibres in the manufacture of a food product, a dietary supplement or a pharmaceutically acceptable composition for increasing the lean body mass.

The bacterium may be used in any form capable of exerting the effects described herein. For example, the bacteria may be viable, dormant, inactivated or dead bacteria. Preferably, the bacteria are viable bacteria.

The bacteria may comprise whole bacteria or may comprise bacterial components. Examples of such components include bacterial cell wall components such as peptidoglycan, bacterial nucleic acids such as DNA and RNA, bacterial membrane components, and bacterial structural components such as proteins, carbohydrates, lipids and combinations of these such as lipoproteins, glycolipids and glycoproteins.

The bacteria may also or alternatively comprise bacterial metabolites. In the present specification the term "bacterial metabolites" includes all molecules produced or modified by the (probiotic) bacteria as a result of bacterial metabolism during growth, survival, persistence, transit or existence of bacteria during the manufacture of the probiotic product and storage and during gastrointestinal transit in a mammal. Examples include all organic acids, inorganic acids, bases, proteins and peptides, enzymes and co-enzymes, amino acids and nucleic acids, carbohydrates, lipids, glycoproteins, lipoproteins, glycolipids, vitamins, all bioactive compounds, metabolites containing an inorganic component, and all small molecules, for example nitrous molecules or molecules containing a sulphurous acid.

Preferably the bacteria comprise whole bacteria, more preferably whole viable bacteria.

Preferably, the *Bifidobacterium* used in accordance with the present invention is one which is suitable for human and/or animal consumption. A skilled person will be readily aware of specific species and or strains of *Bifidobacteria* from within the genera described herein which are used in the food and/or agricultural industries and which are generally considered suitable for human and/or animal consumption.

Disclosed herein are the species *Bifidobacterium lactis, Bifidobacterium bifidium, Bifidobacterium longum, Bifidobacterium animalis, Bifidobacterium breve, Bifidobacterium infantis, Bifidobacterium catenulatum, Bifidobacterium pseudocatenulatum, Bifidobacterium adolescentis, and Bifidobacterium angulatum,* and combinations of any thereof.

The *Bifidobacterium* used in the present invention is of the species *Bifidobacterium animalis.* More specifically, the *Bifidobacferium* used in the present invention is of the *Bifidobacterium animalis* ssp. *lactis.*

Most specifically, the bacteria used in the present invention is *Bifidobacterium animalis* ssp. *lactis* (strain) 420 (B420). This strain is commercially available from DuPont Nutrition Biosciences ApS.

This strain of *Bifidobacterium animalis* ssp. *lactis* has also been deposited under the reference DGCC420 by DuPont Nutrition Biosciences ApS, of Langebrogade 1, DK-1411 Copenhagen K, Denmark, in accordance with the Budapest Treaty on 30 June 2015 at the Leibniz-Institut Deutsche Sammlung von Mikroorganismen und Zellkulturen GmbH (DSMZ), Inhoffenstrasse 7B, 38124 Braunschweig, Germany, where it is recorded under registration number DSM 32073. It is requested that the biological material shall be made available only by the issue of a sample to an expert nominated by the requester.

In one embodiment, the bacterium used in the present invention is a probiotic bacterium. In this specification the term 'probiotic bacterium' is defined as covering any non-pathogenic bacterium which, when administered live in adequate amounts, confer a health benefit on the host. These probiotic strains generally have the ability to survive the passage through the upper part of the digestive tract. They are non-pathogenic, non-toxic and exercise their beneficial effect on health on the one hand via ecological interactions with the resident flora in the digestive tract, and on the other hand via their ability to influence the immune system in a positive manner via the "GALT" (gut-associated lymphoid tissue). Depending on the definition of probiotics, these bacteria, when given in a sufficient number, have the ability to progress live through the intestine, however they do not cross the intestinal barrier and their primary effects are therefore induced in the lumen and/or the wall of the gastrointestinal tract. They then form part of the resident flora during the administration period. This colonization (or transient colonization) allows the probiotic bacteria to exercise a beneficial effect, such as the repression of potentially pathogenic micro-organisms present in the flora and interactions with the immune system of the intestine.

In preferred embodiments, the bacterium used in the present invention is a probiotic *Bifidobacterium* or a mixture thereof.

### Dosage

The *Bifidobacterium,* such as a strain of *Bifidobacterium animalis* ssp. *lactis,* for example *Bifidobacterium animalis* ssp. *lactis* (strain) 420 (B420), used in accordance with the present invention may comprise from 10⁶ to 10¹² CFU of bacteria/g of support, and more particularly from 10⁸ to 10¹² CFU of bacteria/g of support, preferably 10⁹ to 10¹² CFU/g for the lyophilized form.

Suitably, the *Bifidobacterium,* such as a strain of *Bifidobacterium animalis* ssp. *lactis,* for example *Bifidobacterium animalis* ssp. *lactis* (strain) 420 (B420), may be administered at a dosage of from about 10⁶ to about 10¹² CFU of microorganism/dose, preferably about 10⁸ to about 10¹² CFU of microorganism/dose. By the term "per dose" it is meant that this amount of microorganism is provided to a subject either per day or per intake, preferably per day. For example, if the microorganism is to be administered in a food product, for example in a yoghurt, then the yoghurt will preferably contain from about 10⁸ to 10¹² CFU of the microorganism. Alternatively, however, this amount of microorganism may be split into multiple administrations each consisting of a smaller amount of microbial loading - so long as the overall amount of microorganism received by the subject in any specific time, for instance each 24-hour period, is from about 10⁶ to about 10¹² CFU of microorganism, preferably 10⁸ to about 10¹² CFU of microorganism.

In accordance with the present invention an effective amount of at least one strain of a microorganism may be at least 10⁶ CFU of microorganism/dose, preferably from about 10⁶ to about 10¹² CFU of microorganism/dose, preferably about 10⁸ to about 10¹² CFU of microorganism/dose.

In one embodiment, preferably the *Bifidobacterium,* such as a strain of *Bifidobacterium animalis* ssp. *lactis,* for example *Bifidobacterium animalis* ssp. *lactis* (strain) 420 (B420), may be administered at a dosage of from about 10⁶ to about 10¹² CFU of microorganism/day, preferably about 10⁸ to about 10¹² CFU of microorganism/day. Hence, the effective amount in this embodiment may be from about 10⁶ to about 10¹² CFU of microorganism/day, preferably about 10⁸ to about 10¹² CFU of microorganism/day.

CFU stands for "colony-forming units". By 'support' is meant the food product, dietary supplement or the pharmaceutically acceptable formulation.

In one embodiment, the present invention relates to a composition comprising a bacterium of the genus *Bifidobacterium* or a mixture thereof, and one or more prebiotics and/or fibres, in the form of a food product, a dietary supplement or a pharmaceutically acceptable composition. The composition comprising the bacterium of the genus *Bifidobacterium* or a mixture thereof, such as a strain of *Bifidobacterium animalis* ssp. *lactis,* for example *Bifidobacterium animalis* ssp. *lactis* (strain) 420 (B420), is in the form of a food product, a dietary supplement or a pharmaceutically acceptable composition.

In a further embodiment, the lean body mass is increased in the trunk, legs, android and gynoid regions.

Lean Body Mass is a component of body composition, calculated by subtracting body fat weight from total body weight. Total body weight is lean plus fat. In a mathematical equation: Lean Body Mass (LBM) = Body weight (BW) - Body Fat (BF).

In a still further embodiment, the lean body mass is muscle mass.

By "muscle", we mean a tissue composed of cells capable of contraction and relaxation to produce movement in an organ or part of an organ.

### Effects/Subjects/Medical indications

The composition to which the present invention relates is administered to a mammal, including for example livestock (including cattle, horses, pigs and sheep), and humans. In some aspects of the present invention the mammal is a companion animal (including pets), such as a dog or a cat for instance. In some aspects of the present invention, the subject may suitably be a human.

The composition to which the present invention relates may be suitable for use in increasing lean body mass in mammals.

Although birds and poultry, including chickens, are technically not mammals, the present invention may also be suitable for birds and any type of poultry, such as chickens.

In this specification the term "increasing lean body mass" refers to any administration of the composition according to the present invention and includes increasing lean body mass in mammal, birds or poultry.

In particular, the composition according to the present invention is suitable for mammals, birds and poultry losing lean body mass or which present the need to increase the lean body mass. This aspect is discussed in more detail below.

### Lean Body Mass

As noted above, subject mammals, birds or poultry treated with the composition according to the present invention may increase their lean body mass mitigating the consequences of the loss of the lean body mass.

Adult lean body mass declines with age, in a process known as Sarcopenia. Sarcopenia is an age-related loss in lean body mass accelerated by poor nutrition and physical inactivity. To counteract this loss of muscle, patients should consume protein in adequate quantities and at proper times.

Adult lean body mass also declines with certain diseases, such as kidney diseases.

### Compositions

While is it possible to administer the composition alone according to the present invention (i.e. without any support, diluent or excipient), the composition is typically and preferably administered on or in a support as part of a product, in particular as a component of a food product, a dietary supplement or a pharmaceutical composition or formulation. These products typically contain additional components well known to those skilled in the art.

By "composition" it is understood the combination of 2 or more substances. The substances may be chemical substances or biological substances, such as bacteria, including the substance that has the desired effect.

By "formulation", it is understood the process or composition in which different chemical and/or biological substances, including the substance having the desired effect, are combined to produce a final product. Composition and formulation may be used interchangeably.

Any product which can benefit from the composition may be used in the present invention. These include but are not limited to foods, particularly fruit conserves and dairy foods and dairy food-derived products, and pharmaceutical products.

### Food

In one embodiment, the composition according to the invention is employed in a food product, such as a food supplement, a drink or a powder based on milk. Here, the term "food" is used in a broad sense and covers food for humans as well as food for animals (i.e. a feed). In a preferred aspect, the food is for human consumption.

The food may be in the form of a solution or as a solid, depending on the use and/or the mode of application and/or the mode of administration.

When used as, or in the preparation of, a food, such as functional food, the composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

By way of example, the composition of the present invention can be used as an ingredient to soft drinks, a fruit juice or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods, balanced foods and drinks, fruit fillings, care glaze, chocolate bakery filling, cheese cake flavoured filling, fruit flavoured cake filling, cake and doughnut icing, instant bakery filling creams, fillings for cookies, ready-to-use bakery filling, reduced calorie filling, adult nutritional beverage, acidified soy/juice beverage, aseptic/retorted chocolate drink, bar mixes, beverage powders, calcium fortified soy/plain and chocolate milk, calcium fortified coffee beverage.

The composition can further be used as an ingredient in food products such as American cheese sauce, anti-caking agent for grated & shredded cheese, chip dip, cream cheese, dry blended whip topping fat free sour cream, freeze/thaw dairy whipping cream, freeze/thaw stable whipped topping, low fat and light natural cheddar cheese, low fat Swiss style yoghurt, aerated frozen desserts, hard pack ice cream, label friendly, improved economics & indulgence of hard pack ice cream, low fat ice cream: soft serve, barbecue sauce, cheese dip sauce, cottage cheese dressing, dry mix Alfredo sauce, mix cheese sauce, dry mix tomato sauce and others.

The term "dairy product" as used herein is meant to include a medium comprising milk of animal and/or vegetable origin. As milk of animal origin there can be mentioned cow's, sheep's, goat's or buffalo's milk. As milk of vegetable origin there can be mentioned any fermentable substance of vegetable origin which can be used according to the invention, in particular originating from soybeans, rice or cereals.

Still more preferably the food product employed according to the invention is a fermented milk or humanized milk.

For certain aspects, preferably the present invention may be used in connection with yoghurt production, such as fermented yoghurt drink, yoghurt, drinking yoghurt, cheese, fermented cream, milk based desserts and others.

Suitably, the composition can be further used as an ingredient in one or more of cheese applications, meat applications, or applications comprising protective cultures.

The present invention also provides a method of preparing a food or a food ingredient, the method comprising admixing the composition according to the present invention with another food ingredient.

Advantageously, the present invention relates to products that have been contacted with the composition of the present invention, and optionally with other components/ingredients, wherein the composition is used in an amount to be capable of improving the nutrition and/or health benefits of the product.

As used herein the term "contacted" refers to the indirect or direct application of the composition of the present invention to the product. Examples of the application methods which may be used, include, but are not limited to, treating the product in a material comprising the composition, direct application by mixing the composition with the product, spraying the composition onto the product surface or dipping the product into a preparation of the composition.

Where the product of the invention is a foodstuff, the composition of the present invention is preferably admixed with the product. Alternatively, the composition may be included in the emulsion or raw ingredients of a foodstuff. In a further alternative, the composition may be applied as a seasoning, glaze, colorant mixture, and the like.

For some applications, it is important that the composition is made available on or to the surface of a product to be affected/treated. This allows the composition to impart one or more of the following favourable characteristics: nutrition and/or health benefits.

The compositions of the present invention may be applied to intersperse, coat and/or impregnate a product with a controlled amount of a microorganism.

Preferably, the composition is used to ferment milk or sucrose fortified milk or lactic media with sucrose and/or maltose where the resulting media containing all components of the composition - i.e. said microorganism according to the present invention - can be added as an ingredient to yoghurt milk in suitable concentrations - such as for example in concentrations in the final product which offer a daily dose of 10⁶ - 10¹⁰ CFU. The microorganism according to the present invention may be used before or after fermentation of the yoghurt.

For some aspects the composition according to the present invention is used as, or in the preparation of, animal feeds, such as livestock feeds, in particular poultry (such as chicken) feed, or pet food.

Advantageously, where the product is a food product, the composition comprising the bacterium of the genus *Bifidobacterium* or a mixture thereof, and one or more prebiotics and/or fibres, should remain effective through the normal "sell-by" or "expiration" date during which the food product is offered for sale by the retailer. Preferably, the effective time should extend past such dates until the end of the normal freshness period when food spoilage becomes apparent. The desired lengths of time and normal shelf life will vary from foodstuff to foodstuff and those of ordinary skill in the art will recognise that shelf-life times will vary upon the type of foodstuff, the size of the foodstuff, storage temperatures, processing conditions, packaging material and packaging equipment.

### Food ingredient

The composition of the present invention may be used as a food ingredient and/or feed ingredient.

As used herein the term "food ingredient" or "feed ingredient" includes a formulation which is or can be added to functional foods or foodstuffs as a nutritional supplement.

The food ingredient may be in the form of a solution or as a solid, depending on the use and/or the mode of application and/or the mode of administration.

### Food Supplements

The composition of the present invention may be - or may be added to - dietary supplements, also referred to herein as food supplements.

Here, the term "dietary supplement" is a product intended for ingestion that contains a "dietary ingredient" intended to add further nutritional value to (supplement) the diet. A "dietary ingredient" may be one, or any combination, of the following substances: a vitamin, a mineral, an herb or other botanical, an amino acid, a dietary substance for use by people to supplement the diet by increasing the total dietary intake, a concentrate, metabolite, constituent, or extract.

Dietary supplements may be found in many forms such as tablets, capsules, soft gels, gel caps, liquids, or powders. Some dietary supplements can help ensure that you get an adequate dietary intake of essential nutrients; others may help you reduce your risk of disease.

### Functional Foods

The composition of the present invention may be - or may be added to - functional foods.

As used herein, the term "functional food" means food which is capable of providing not only a nutritional effect, but is also capable of delivering a further beneficial effect to consumer.

Accordingly, functional foods are ordinary foods that have components or ingredients (such as those described herein) incorporated into them that impart to the food a specific functional - e.g. medical or physiological benefit - other than a purely nutritional effect.

Although there is no legal definition of a functional food, most of the parties with an interest in this area agree that they are foods marketed as having specific health effects beyond basic nutritional effects.

Some functional foods are nutraceuticals. Here, the term "nutraceutical" means a food which is capable of providing not only a nutritional effect and/or a taste satisfaction, but is also capable of delivering a therapeutic (or other beneficial) effect to the consumer. Nutraceuticals cross the traditional dividing lines between foods and medicine.

### Medical Food

In one embodiment, the composition of the present invention is in the form of a medical food. Preferably, the composition of the present invention comprising a strain of *Bifidobacterium animalis* ssp. *lactis,* for example *Bifidobacterium animalis* ssp. *lactis* (strain) 420 (B420) is in the form of a medical food.

By "medical food" it is meant a food which is formulated to be consumed or administered with or without the supervision of a physician and which is intended for a specific dietary management or condition for which distinctive nutritional requirements, based on recognized scientific principles, are established by medical evaluation.

### Pharmaceutical

The composition of the present invention may be used as - or in the preparation of - a pharmaceutical formulation or composition. Here, the term "pharmaceutical" is used in a broad sense - and covers pharmaceuticals for humans as well as pharmaceuticals for animals (i.e. veterinary applications). In a preferred aspect, the pharmaceutical is for human use and/or for animal husbandry.

The pharmaceutical can be for therapeutic purposes - which may be curative or palliative or preventative in nature. The pharmaceutical may even be for diagnostic purposes.

A pharmaceutically acceptable formulation or support or composition may be for example a formulation or support in the form of compressed tablets, tablets, capsules, ointments, suppositories or drinkable solutions. Other suitable forms are provided below.

When used as - or in the preparation of - a pharmaceutical, the composition of the present invention may be used in conjunction with one or more of: a pharmaceutically acceptable carrier, a pharmaceutically acceptable diluent, a pharmaceutically acceptable excipient, a pharmaceutically acceptable adjuvant, a pharmaceutically active ingredient.

The pharmaceutical may be in the form of a solution or as a solid - depending on the use and/or the mode of application and/or the mode of administration.

The composition of the present invention may be used as pharmaceutical ingredients. Here, the composition may be the sole active component or it may be at least one of a number (i.e. 2 or more) of active components.

The pharmaceutical ingredient may be in the form of a solution or as a solid - depending on the use and/or the mode of application and/or the mode of administration.

The composition may be used according to the present invention in any suitable form - whether when alone or when present in a combination with other components or ingredients. Combinations comprising the composition of the present invention and other components and/or ingredients (i.e. ingredients - such as food ingredients, functional food ingredients or pharmaceutical ingredients) may be used in any suitable form.

The composition may be used according to the present invention in the form of solid or liquid preparations or alternatives thereof. Examples of solid preparations include, but are not limited to tablets, capsules, dusts, granules and powders which may be wettable, spray-dried or freeze-dried. Examples of liquid preparations include, but are not limited to, aqueous, organic or aqueous-organic solutions, suspensions and emulsions.

Suitable examples of forms include one or more of: tablets, pills, capsules, ovules, solutions or suspensions, which may contain flavouring or colouring agents, for immediate-, delayed-, modified-, sustained-, pulsed- or controlled-release applications.

By way of example, if the composition of the present invention is used in a tablet form - such for use as a functional ingredient - the tablets may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethrai fatty acid esters, hydroxymethylceilulose, polyvinylpyrrolidone, and the like.

Preferred excipients for the forms include lactose, starch, a cellulose, milk sugar or high molecular weight polyethylene glycols.

For aqueous suspensions and/or elixirs, the composition of the present invention may be combined with various sweetening or flavouring agents, colouring matter or dyes, with emulsifying and/or suspending agents and with diluents such as water, propylene glycol and glycerin, and combinations thereof.

The forms may also include gelatin capsules; fibre capsules, fibre tablets etc.; or even fibre beverages.

Further examples of form include creams. For some aspects the microorganism used in the present invention may be used in pharmaceutical and/or cosmetic creams such as sun creams and/or after-sun creams for example.

In one aspect, the composition according to the present invention may be administered in an aerosol, for example by way of a nasal spray, for instance for administration to the respiratory tract.

### Medicament

In one embodiment, the composition of the present invention is in the form of a medicament.

The term "medicament" as used herein encompasses medicaments for both human and animal usage in human and veterinary medicine. In addition, the term "medicament" as used herein means any substance which provides a therapeutic and/or beneficial effect. The term "medicament" as used herein is not necessarily limited to substances which need Marketing Approval, but may include substances which can be used in cosmetics, nutraceuticals, food (including feeds and beverages for example), probiotic cultures, and natural remedies. In addition, the term "medicament" as used herein encompasses a product designed for incorporation in animal feed, for example livestock feed and/or pet food.

### Prebiotics

In one embodiment, the composition of the present invention comprises a bacterium of the genus *Bifidobacterium* or a mixture thereof, and one or more prebiotics and/or fibres.

Prebiotics are a category of functional food, defined as non-digestible food ingredients that beneficially affect the host by selectively stimulating the growth and/or activity of one or a limited number of bacteria (particularly, although not exclusively, probiotics, *Bifidobacteria* and/or lactic acid bacteria) in the colon, and thus improve host health. Typically, prebiotics are carbohydrates (such as oligosaccharides), but the definition does not preclude non-carbohydrates. The most prevalent forms of prebiotics are nutritionally classed as soluble fibres. To some extent, many forms of dietary fibres exhibit some level of prebiotic effect.

In one embodiment, a prebiotic is a selectively fermented ingredient that allows specific changes, both in the composition and/or activity in the gastrointestinal microflora that confers benefits upon host well-being and health.

Suitably, the prebiotic may be used according to the present invention in an amount of 0.01 to 100 g/day, preferably 0.1 to 50 g/day, more preferably 0.5 to 20 g/day. In one embodiment, the prebiotic may be used according to the present invention in an amount of 1 to 10 g/day, preferably 2 to 9 g/day, more preferably 3 to 8 g/day. In another embodiment, the prebiotic may be used according to the present invention in an amount of 5 to 50 g/day, preferably 10 to 25 g/day.

Examples of dietary sources of prebiotics include soybeans, inulin sources (such as Jerusalem artichoke, jicama, and chicory root), raw oats, unrefined wheat, unrefined barley and yacon.

Examples of suitable prebiotics include alginate, xanthan, pectin, locust bean gum (LBG), inulin, guar gum, galacto-oligosaccharide (GOS), fructo-oligosaccharide (FOS), polydextrose (i.e. Litesse^{®}), lactitol, lactosucrose, soybean oligosaccharides, isomaltulose (Palatinose TM), isomalto-oligosaccharides, gluco-oligosaccharides, xylooligosaccharides, manno-oligosaccharides, beta-glucans, cellobiose, raffinose, gentiobiose, melibiose, xylobiose, cyciodextrins, isomaltose, trehalose, stachyose, panose, pullulan, verbascose, galactomannans, and all forms of resistant starches.

A particularly preferred example of a prebiotic is polydextrose.

The combination of *Bifidobacterium* and one or more prebiotics and/or fibres present in the composition according to the present invention exhibits a synergistic effect (i.e. an effect which is greater than the additive effect of the bacteria when used separately). Without wishing to be bound by theory, it is believed that such a combination is capable of selectively stimulating the growth and/or activity of the *Bifidobacteria* in the colon, and thus improving its effect and the host health.

The *Bifidobacterium* or a mixture thereof used in the combination with one or more prebiotics and/or fibres is of the *Bifidobacterium animalis* ssp. *lactis* (strain) 420 (B420).

Suitably, the fibre and/or prebiotic used in the combination is polydextrose.

In another embodiment, the fibre and/or prebiotic used in the combination is Litesse^{®}.

In a further aspect, the invention comprises a food product containing a composition comprising the *Bifidobacterium* or mixture thereof and one or more prebiotics and/or fibres, wherein the *Bifidobacterium* or mixture thereof is of the species *Bifidobacterium animalis lactis* (strain) 420 (B420).

In a further aspect, the invention comprises a dietary supplement containing a composition comprising a *Bifidobacterium* or mixture thereof and one or more prebiotics and/or fibres, wherein the *Bifidobacterium* or mixture thereof is of the *Bifidobacterium animalis* ssp. *lactis* (strain) 420 (B420).

In a further aspect, the invention comprises a pharmaceutically acceptable composition containing a composition comprising a *Bifidobacterium* or mixture thereof and one or more prebiotics and/or fibres, wherein the *Bifidobacterium* or mixture thereof is of the *Bifidobacterium animalis* ssp. *lactis* (strain) 420 (B420).

### Examples

### Materials and methods

### Clinical study design and screening criteria

The intervention was a double-blind, randomized, placebo-controlled, multi-centre parallel study, conducted according to Good Clinical Practice and the Declaration of Helsinki.

A cohort of 225 out of 263 overweight and obese adults was recruited at four research centres in southern Finland and randomized according to a 1:1:1:1 allocation to one of four groups:
1) Placebo (microcrystalline cellulose), 12 g/day;
2) Polydextrose (Pdx), 12 g/day;
3) Probiotic B420 (Bifidobacterium animalis ssp. lactis (Strain) 420), 10¹⁰ CFU/day; or
4) B420 and Pdx, 10¹⁰ CFU/day + 12 g/day.

The products for the study were provided in a sachet that the participant mixed with a 250 ml fruit smoothie once per day at the time of their liking for six months.

All randomized participants were 18-65 years old with a Body Mass Index (BMI, calculated as body weight [kg] divided by height [m] squared) between 28.0-34.9 and a waist-hip ratio of ≥0.88 for males and ≥0.83 for females. The most important exclusion criteria included diagnosed metabolic diseases or the use of related medications; use of laxatives, fibre supplements or probiotics in the previous 6 weeks; history of bariatric surgery; use of antiobesity drugs in the previous 3 months, recent (past 2 months) or on-going use of antimicrobials; on-going or recent participation in a weight-loss program; weight change of 3 kg during previous 3 months; and pregnancy.

### Recruitment and study populations

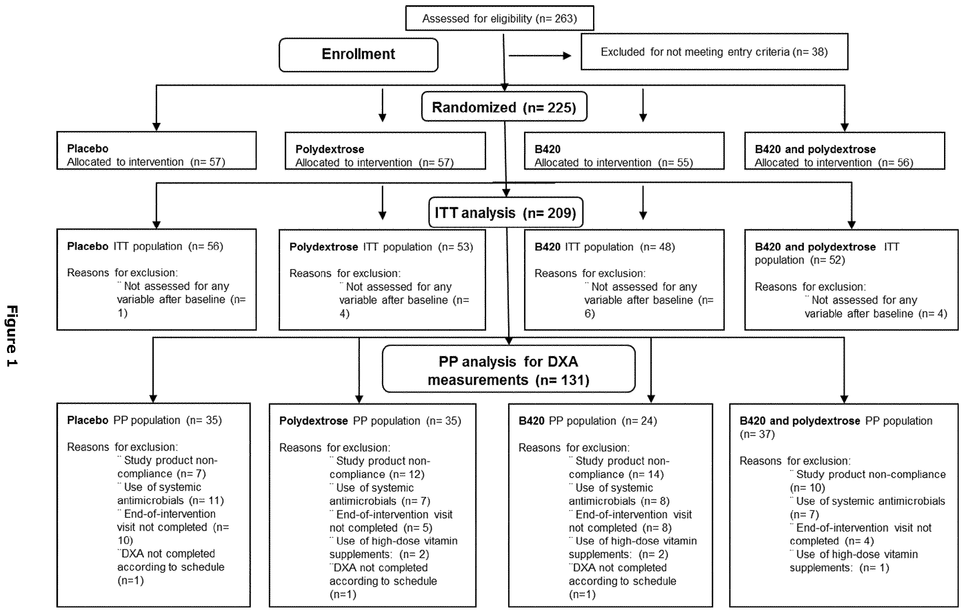

Before unblinding the study, the 225 participants were divided into an Intention-to-Treat (ITT) and a Per Protocol (PP) population. The ITT population contained all 209 participants who were assessed for any parameter after the baseline visit. The PP population contained participants who completed the intervention period with at least 80% study product compliance, and did not use systemic antimicrobials or high-dose vitamin supplements during the intervention. In addition, 3 participants were excluded from the Dual-energy X-ray absorptiometry (DXA) analyses because of not completing the DXA measurement according to schedule, i.e. within ten days after the end of treatment, resulting in a PP population of 131 participants. The PP population contained all participants who were considered compliant with the protocol and therefore better represents the actual efficacy of the study product (Figure 1).

Figure 1 shows that before unblinding the study, participants were divided into an Intention-to-Treat (ITT) population and a Per Protocol (PP) population according to adherence to the study protocol (n represents the number of people involved in each step of the process).

### Measurement of lean body mass

The study participants underwent a dual-energy x-ray absorptiometry (DXA) scan at baseline, 2 months, 4 months, 6 months (end-of-intervention) and one month after the end of intervention (follow-up +1 months). DXA scans were conducted at private medical centers by trained staff. The device automatically calculates body fat mass, bone mass and lean body mass from the x-ray measurement. Therefore, lean body mass mostly consists of muscle mass, internal organ mass and water. Results were collected separately for total lean body mass and lean body mass in the individual regions of the body (arms, legs, trunk, android [waist] and gynoid [hip] regions).

### Statistical analysis

The mean relative change from baseline in all three active groups:
- groups taking B420 alone;
- groups taking B420 and polydextrose (Pdx) combined; and
- polydextrose (Pdx) alone,
was compared to placebo as an overall effect (one-way analysis of covariance, using baseline values as covariate).

The three active groups were then compared to placebo separately using Dunnett's test, which corrects for multiple comparisons. All analyses were conducted with SAS analysis software, version 9.3, using relative changes from baseline to the end of treatment (six months). In the ITT population, missing observations were handled with the Last Observation Carried Forward method. In statistics, Dunnett's test is a multiple comparison procedure developed by Canadian statistician Charles Dunnett to compare each of a number of treatments with a single control. Multiple comparisons to a control are also referred to as many-to-one comparisons. The Last Observation Carried Forward method means that for missing values the latest measured value from a previous time point was used in the analysis. A P-value below 0.05 was considered statistically significant, meaning that the hypothesis of the compared observations being different is true with a 95% probability. A P-value above 0.05 does not prove that there was no difference, but rather there is not enough statistical power to draw conclusions with confidence.

### Results

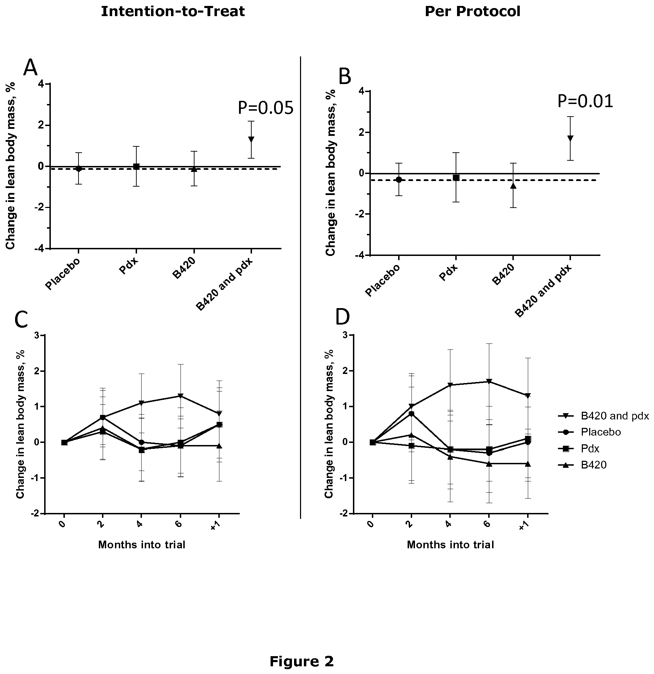

There was a statistically significant increase in lean body mass in the group taking B420 and Pdx combined when compared with placebo in the PP population (P=0.012), and a borderline non-significant difference in the ITT population (P=0.05) (Figures 2A-B).

The overall differences between active and placebo were not significant (ITT: P=0.28; PP: P=0.30), most likely due to the lack of any effect in the groups taking B420 alone and Pdx alone (Figure 2A-B). The change in lean mass in the group taking B420 and Pdx combined happened gradually during the course of the study (Figures 2C-D).

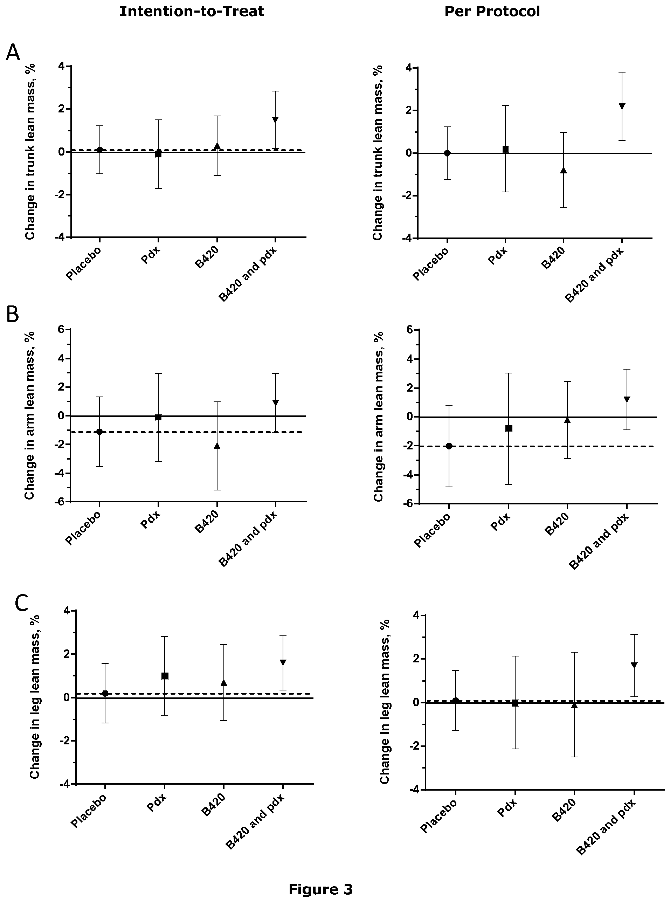

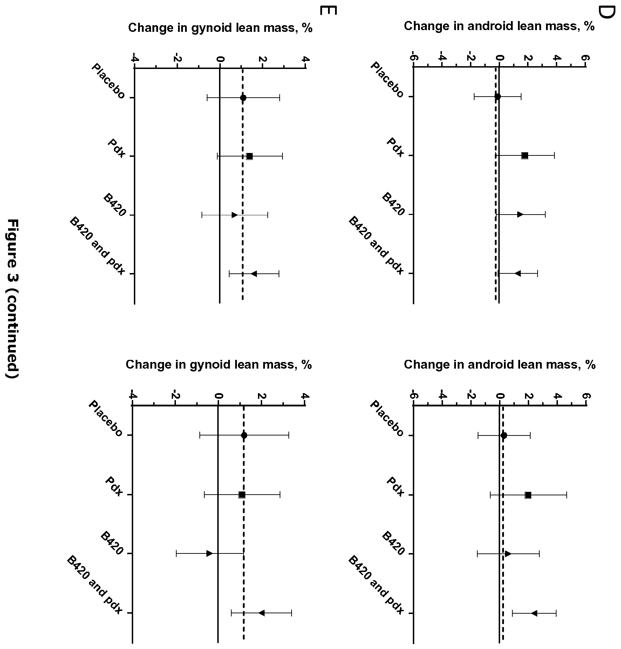

There were no statistically significant differences between active and placebo in the individual regions of the body, although in all regions the group taking B420 and Pdx combined was the only one showing a significant increase in lean body mass compared to baseline (Figure 3). The differences between B420 and Pdx combined vs Placebo were most pronounced in the trunk (Figure 3A) and leg (Figure 3C) regions in the protocol-compliant PP population. Android (waist) lean mass did not show a similar difference between B420 and Pdx combined vs Placebo, which suggests that the increase in lean mass was not due to any abdominal oedema, but rather an actual increase in muscle mass, which is also supported by the increase in lean mass in the leg region where lean tissue mass mostly consists of muscle mass.

Figure 2 shows the relative changes in total lean body mass in the Intention-to-Treat (A) and Per Protocol (B) populations, as well as development of lean body mass during the study in the Intention-to-Treat (C) and Per Protocol (D) populations. P-values below 0.05 in panels A and B signify statistically significant differences from placebo. Results are displayed as mean +/- 95% confidence interval (CI). When the 95% CI does not overlap with the baseline (solid line), it indicates a significant change from baseline within that group.

Figure 3 shows the relative changes in trunk (A), arms (B), legs (C) and the android (D) and gynoid (E) regions from baseline to six months of treatment in the Intention-to-Treat and Per Protocol populations. P-values below 0.05 mark significant differences from Placebo. Results are displayed as mean +/- 95% confidence interval (CI). When the 95% CI does not overlap with the baseline (solid line), it indicates a significant change from baseline within that group.

Absolute values of lean mass at the beginning and end of the intervention period at the different regions of the body are shown in Table 1.

**Table 1. Lean body mass in the different regions of the body per group.**

| | | | **Placebo** | **Pdx** | **B420** | **B420 and Pdx** |
|---|---|---|---|---|---|---|
| | | | *Mean*±*SD* | *Mean*±*SD* | *Mean*±*SD* | *Mean*±*SD* |
| **Intention-to-Treat** | *Visit* | ***n*** | 48-55* | 47-53* | 39-48* | 48-52* |
| Total lean body mass [kg] | *Baseline* | | 47.90 ± 9.21 | 49.31 ± 9.73 | 48.53 ± 9.13 | 46.63 ± 9.31 |
| | 6 *months* | | 48.78 ± 9.56 | 49.25 ± 9.50 | 49.10 ± 9.72 | 46.72 ± 9.30 |
| Trunk lean body mass [kg] | *Baseline* | | 22.82 ± 4.31 | 23.56 ± 4.40 | 23.04 ± 3.84 | 22.31 ± 3.97 |
| | 6 *months* | | 23.34 ± 4.38 | 23.49 ± 4.38 | 23.39 ± 4.18 | 22.45 ± 4.08 |
| Arm lean body mass [kg] | *Baseline* | | 5.54 ± 1.61 | 5.69 ± 1.72 | 5.73 ± 1.69 | 5.32 ± 1.62 |
| | 6 *months* | | 5.62 ± 1.71 | 5.69 ± 1.74 | 5.67 ± 1.78 | 5.23 ± 1.53 |
| Leg lean body mass [kg] | *Baseline* | | 16.23 ± 3.22 | 16.63 ± 3.49 | 16.36 ± 3.52 | 15.60 ± 3.57 |
| | 6 *months* | | 16.49 ± 3.43 | 16.68 ± 3.30 | 16.67 ± 3.57 | 15.67 ± 3.64 |
| Android lean body mass [kg] | *Baseline* | | 3.41 ± 0.70 | 3.51 ± 0.66 | 3.43 ± 0.63 | 3.31 ± 0.61 |
| | 6 *months* | | 3.48 ± 0.68 | 3.56 ± 0.68 | 3.52 ± 0.61 | 3.32 ± 0.61 |
| Gynoid lean body mass [kg] | *Baseline* | | 7.01 ± 1.50 | 7.20 ± 1.48 | 7.12 ± 1.50 | 6.81 ± 1.43 |
| | 6 *months* | | 7.21 ± 1.52 | 7.29 ± 1.47 | 7.22 ± 1.44 | 6.84 ± 1.36 |

| **Per-Protocol** | | ***n*** | **35** | **35** | **24** | **37** |
|---|---|---|---|---|---|---|
| Total lean body mass [kg] | *Baseline* | | 49.28 ± 9.43 | 50.01 ± 9.44 | 49.13 ± 9.42 | 46.23 ± 9.51 |
| | 6 *months* | | 49.13 ± 9.59 | 49.88 ± 9.41 | 48.86 ± 9.50 | 47.05 ± 9.96 |
| Trunk lean body mass [kg] | *Baseline* | | 23.44 ± 4.40 | 23.78 ± 4.42 | 23.37 ± 4.17 | 22.13 ± 4.09 |
| | 6 *months* | | 23.43 ± 4.39 | 23.77 ± 4.28 | 23.16 ± 4.06 | 22.62 ± 4.37 |
| Arm lean body mass [kg] | *Baseline* | | 5.79 ± 1.70 | 5.81 ± 1.68 | 5.76 ± 1.59 | 5.22 ± 1.55 |
| | 6 *months* | | 5.68 ± 1.77 | 5.76 ± 1.79 | 5.74 ± 1.63 | 5.28 ± 1.64 |
| Leg lean body mass [kg] | *Baseline* | | 16.68 ± 3.22 | 16.97 ± 3.33 | 16.58 ± 3.49 | 15.49 ± 3.71 |
| | 6 *months* | | 16.70 ± 3.35 | 16.95 ± 3.32 | 16.57 ± 3.66 | 15.77 ± 3.90 |
| Android lean body mass [kg] | *Baseline* | | 3.50 ± 0.70 | 3.52 ± 0.67 | 3.47 ± 0.68 | 3.26 ± 0.63 |
| | 6 *months* | | 3.51 ± 0.68 | 3.58 ± 0.68 | 3.47 ± 0.63 | 3.34 ± 0.64 |
| Gynoid lean body mass [kg] | *Baseline* | | 7.22 ± 1.47 | 7.33 ± 1.44 | 7.17 ± 1.50 | 6.74 ± 1.46 |
| | 6 *months* | | 7.29 ± 1.44 | 7.39 ± 1.41 | 7.13 ± 1.46 | 6.87 ± 1.46 |

| | | | | | | |
|---|---|---|---|---|---|---|
| *Number of participants (n) at 6-month visit (lower value) and baseline visit (higher value). Mean values at baseline in the Intention-to-Treat population include participants who withdrew from the study before 6 months, and hence changes from baseline do not directly translate to the changes from baseline as shown in Figures 2-3. However, all values are included in the statistical analyses (Last Observation Carried Forward technique, as explained earlier); SD= Standard Deviation. | | | | | | |

## Claims

1. A composition comprising a bacterium of the genus *Bifidobacterium* or a mixture thereof, and one or more prebiotics and/or fibres, for therapeutic use in treating and preventing sarcopenia by increasing lean body mass in a mammal, wherein the bacterium of the genus *Bifidobacterium* is *Bifidobacterium* animalis ssp. *lactis* (strain) 420 (B420).

2. The composition for the therapeutic use according to claim 1, wherein the *Bifidobacterium* is a probiotic *Bifidobacterium* or a mixture thereof.

3. The composition for the therapeutic use according to claim 1, wherein the prebiotics and/or fibres is polydextrose.

4. The composition for the therapeutic use according to any one of the preceding claims, wherein the composition is in the form of a food product, a dietary supplement or a pharmaceutically acceptable composition.

5. A non-therapeutic use of a composition comprising a bacterium of the genus *Bifidobacterium* or a mixture thereof, and one or more prebiotics and/or fibres for increasing lean body mass in a mammal, wherein the bacterium of the genus *Bifidobacterium* is *Bifidobacterium* animalis ssp. *lactis* (strain) 420 (B420).

6. The non-therapeutic use according to claim 5, wherein the fibres and/or prebiotics is polydextrose.

7. The non-therapeutic use according to claims 5-6, wherein the composition is in the form of a food product, a dietary supplement or a pharmaceutically acceptable composition.

## Patentansprüche

1. Zusammensetzung, umfassend ein Bakterium der Gattung *Bifidobacterium* oder ein Gemisch davon sowie ein/einen oder mehrere Präbiotika und/oder Ballaststoffe, zur therapeutischen Verwendung bei der Behandlung oder Vorbeugung von Sarkopenie durch Erhöhen der fettfreien Körpermasse bei einem Säuger, wobei es sich bei dem Bakterium der Gattung *Bifidobacterium* um *Bifidobacterium* animalis ssp. *lactis* (Stamm) 420 (B420) handelt.

2. Zusammensetzung zur therapeutischen Verwendung nach Anspruch 1, wobei es sich bei dem *Bifidobacterium* um ein probiotisches *Bifidobacterium* oder ein Gemisch davon handelt.

3. Zusammensetzung zur therapeutischen Verwendung nach Anspruch 1, wobei es sich bei den Präbiotika und/oder Ballaststoffen um Polydextrose handelt.

4. Zusammensetzung zur therapeutischen Verwendung nach einem der vorhergehenden Ansprüche, wobei die Zusammensetzung in Form eines Lebensmittelprodukts, eines Nahrungsergänzungsmittels oder einer pharmazeutisch unbedenklichen Zusammensetzung vorliegt.

5. Nicht therapeutische Verwendung einer ein Bakterium der Gattung *Bifidobacterium* oder ein Gemisch davon sowie ein/einen oder mehrere Präbiotika und/oder Ballaststoffe umfassenden Zusammensetzung zum Erhöhen der fettfreien Körpermasse bei einem Säuger, wobei es sich bei dem Bakterium der Gattung *Bifidobacterium* um *Bifidobacterium* animalis ssp. *lactis* (Stamm) 420 (B420) handelt.

6. Nicht therapeutische Verwendung nach Anspruch 5, wobei es sich bei den Ballaststoffen und/oder Präbiotika um Polydextrose handelt.

7. Nicht therapeutische Verwendung nach Anspruch 5-6, wobei die Zusammensetzung in Form eines Lebensmittelprodukts, eines Nahrungsergänzungsmittels oder einer pharmazeutisch unbedenklichen Zusammensetzung vorliegt.

## Revendications

1. Composition comprenant une bactérie du genre *Bifidobacterium* ou un mélange correspondant, et un ou plusieurs prébiotiques et/ou fibres, pour une utilisation thérapeutique dans le traitement et la prévention de la sarcopénie en augmentant la masse corporelle maigre chez un mammifère, la bactérie du genre *Bifidobacterium* étant *Bifidobacterium animalis* ssp. *lactis* (souche) 420 (B420).

2. Composition pour l'utilisation thérapeutique selon la revendication 1, le *Bifidobacterium* étant un *Bifidobacterium* probiotique ou un mélange correspondant.

3. Composition pour l'utilisation thérapeutique selon la revendication 1, les prébiotiques et/ou fibres étant un polydextrose.

4. Composition pour l'utilisation thérapeutique selon l'une quelconque des revendications précédentes, la composition étant sous la forme d'un produit alimentaire, d'un supplément diététique ou d'une composition pharmaceutiquement acceptable.

5. Utilisation non thérapeutique d'une composition comprenant une bactérie du genre *Bifidobacterium* ou un mélange correspondant, et un ou plusieurs prébiotiques et/ou fibres pour l'augmentation de la masse corporelle maigre chez un mammifère, la bactérie du genre *Bifidobacterium* étant *Bifidobacterium animalis* ssp. *lactis* (souche) 420 (B420).

6. Utilisation non thérapeutique selon la revendication 5, les prébiotiques et/ou fibres étant un polydextrose.

7. Utilisation non thérapeutique selon les revendications 5 et 6, la composition étant sous la forme d'un produit alimentaire, d'un supplément diététique ou d'une composition pharmaceutiquement acceptable.
